# EUROPEAN PATENT APPLICATION

(11) **EP 0 844 482 A2**
(43) Date of publication of application: **27.05.1998**
(21) Application number: 97120380.7
(22) Date of filing: 20.11.1997
(51) Int. Cl.: G01N 33/50, G01N 15/04, G01N 15/05

(54) **Process for enhancing the aggregation and/or agglutination of erythrocytes prior to centrifugation**

(30) Priority: 22.11.1996 US 755301
(71) Applicant: Wardlaw, Stephen Clark, Old Saybrook, CT 06475 (US); Levine, Robert Aaron, Guilford, Connecticut 06437 (US)
(72) Inventor: Wardlaw, Stephen Clark, Old Saybrook, CT 06475 (US); Levine, Robert Aaron, Guilford, Connecticut 06437 (US)
(74) Representative: Klunker . Schmitt-Nilson . Hirsch

(57) **Abstract**

A sample of anticoagulated mammalian whole blood is admixed with a combination of reagents that will reduce the natural repulsive forces that mammalian erythrocytes have for each other. The treated blood sample is then centrifuged in a tube containing a buffy coat expanding insert thereby physically expanding the axial extent of the blood sample 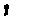 s buffy coat components in the tube. By reducing the tendency of the erythrocytes to repel each other, a clearer demarcation between the erythrocytes and the buffy coat can be achieved. The effect of agglutinating reagents which may have been added to the blood sample will also be enhanced. The aforesaid procedure and reagents make it possible for the first time to accurately analyze a centrifuged sample of anticoagulated bovine whole blood and obtain hematocrit and differential white cell counts therefrom. Additionally, human blood samples which otherwise exhibit a streaming tendency can also be accurately analyzed by the addition of a combination of the appropriate erythrocyte repulsion-reducing reagents along with agglutinating reagents.

## Description

### Technical Field

This invention relates to a process for lowering the natural tendency of erythrocytes to repel each other. More particularly, this invention relates to a process for clarifying the interface between the erythrocyte layer and the granulocyte layer of the buffy coat in a centrifuged sample of anticoagulated whole blood.

### Background Art

U.S. Patent Nos. 4,027,660; 4,082,085; 4,137,755; and 5,086,784 relate to an apparatus and methodology for physically elongating blood constituent layers in a centrifuged blood sample contained in a transparent tube, such as a capillary tube. Blood cell parameters are then determined in instruments such as those disclosed in U.S. Patent Nos. 4,156,570 and 4,558,947. This technology has been employed in veterinary medicine, as described in U.S. Patent No. 4,779,976. The aforesaid technology can be conveniently described as the quantitative buffy coat analysis ("q.b.c.a.") technology.

One problem that has been encountered in connection with the aforesaid q.b.c.a. technology relates to a phenomenon which has been termed "streaming" by the developers of this technology. The problem of streaming is partially the result of erythrocyte layer constituents rising up into the granulocyte layer of the buffy coat; and partially the result of granulocytes sinking down into the upper end of the centrifuged erythrocyte layer, both of which are due to an overlapping density of a fraction of the erythrocytes and the granulocytes.

Several solutions have been devised in order to attempt to minimize streaming. One solution involves the use of an additive such as potassium oxalate, which decreases the water content of the erythrocytes and, therefore, increases the density of the erythrocytes. This solution is described in U.S. Patents Nos. 4,159,896 and 4,181,609. This solution, however, does not completely eliminate the problem of streaming.

Another attempt to solve the streaming problem is discussed in U.S. Patent No. 4,695,553. This solution suggests that the blood sample should be centrifuged first in one direction in the tube, and then the tube is reversed in the centrifuge so that the blood sample can subsequently be centrifuged in the opposite direction in the tube. The reasoning behind this approach is that during the first centrifugation step, the lightest erythrocytes will settle on the top of the packed erythrocyte layer; and when the sample is re-centrifuged in the opposite direction, the lightest erythrocytes will be brought into contact with the overlying heavier erythrocytes and will thus not be likely to end up at the granulocyte interface. This solution works, but is cumbersome, and it requires extensive technician intercession.

A third solution to the streaming problem involves the addition to the blood sample of antibodies or other binding agents which are specific to surface antigens on erythrocytes. A suitable antibody is a species-specific anti-glycophorin antibody. The antibodies are operable to attach adjacent erythrocytes to each other, and thus promote erythrocyte agglutination. U.S. Patents Nos. 4,594,165 and 4,940,668 describe the use of erythrocyte agglutination-promoting antibodies in connection with the aforesaid q.b.c.a. technology. In order for the antibodies to perform their intended function, however, the individual erythrocyte cells must first be able to come into relatively close proximity with each other prior to the centrifugation step so that an antibody may bind adjacent erythrocytes to each other.

A factor which further complicates the analysis of anticoagulated whole blood samples by means of the q.b.c.a. procedure, and particularly by employing erythrocyte-specific antibodies, is the natural tendency of erythrocytes to repel each other. This repelling phenomenon is the result of the "zeta potential", which is due to a negative charge that exists on the surface of the erythrocytes resulting from sialic acid residues present on the erythrocyte membranes.

Erythrocytes in a blood sample also have a tendency to aggregate and form what has been referred to as "rouleaux", or "stacks of coins". This rouleauxing tendency is a contributing factor in determining the erythrocyte sedimentation rate of a whole blood sample that is contained in a transparent tube and that is exposed to the influence of gravity. A discussion of the rouleauxing phenomenon is contained in U.S. Patent No. 4,135,819. Counteracting the tendency of erythrocytes to form rouleaux in the blood sample is the aforesaid zeta potential repulsion phenomenon.

U.S. Patent No. 3,902,964, granted September 2, 1975 to D. J. Greenspan describes a method and apparatus for chemically separating plasma or serum from formed elements of blood. This patent suggests the addition of a positively charged polymer such as Polybrene and a hemagglutinin plant extract lectin to a blood sample in order to enhance agglutination of the formed elements in the blood, and the precipitation of these elements from the serum or plasma in the blood. The purpose of the Greenspan invention is to be able to rapidly promote gravimetric separation of the aforesaid blood constituents from each other without centrifuging the blood sample. The Greenspan patent suggests that a single positively charged polymer when combined with a single lectin will be operable to separate the formed elements in a blood sample from the plasma or serum without centrifuging the blood sample. This reference does not discuss any solution to the problem of providing a clearly delineated interface between the erythrocyte layer and the granulocyte layer in a centrifuged sample of anticoagulated whole blood. It has been determined that following the teachings of the aforesaid Greenspan patent does not significantly improve the adverse effects of streaming in centrifuged anticoagulated human whole blood.

It would be desirable to neutralize, or reduce, the zeta potential on erythrocytes in animals and humans to an extent necessary to enhance the tendency of the erythrocytes to aggregate, form rouleaux, and also to agglutinate, so as to improve the aforesaid q.b.c.a. technology in both humans and animals.

### Disclosure of the Invention

This invention relates to a method for enhancing the tendency of erythrocytes in humans and animals, particularly cows, to form rouleaux, or otherwise aggregate. This invention operates by significantly reducing the strength of the repulsion forces created by the erythrocytes 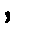 zeta potential. The degree of erythrocyte aggregation; rouleauxing; or agglutination from binding agents present in the subject 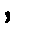 s blood sample, can thus be enhanced to an extent effective to significantly clarify the interface between the erythrocyte layer and the granulocyte layer in a centrifuged anticoagulated whole blood sample.

The method of this invention involves the addition of a combination of reagents to the blood sample which reagents will neutralize or reduce the repulsion effects of the erythrocytes' zeta potential. Applicable reagents include combinations of large molecule cationic macromolecular substances, i.e., those having a molecular weight greater than about 50,000 Daltons. A combination of appropriate reagents in solution will form compound micelles which are useful as an erythrocyte aggregating and rouleaux-promoting agent. Specifically, reagent combinations that may be used include combinations of: polybrene; protamine sulfate; cationic antibiotics such as Vancomycin; polyethylene glycol (PEG); hydroxydiethyl starch (HES); polyvinyl pyrrolidone (PVP); and high molecular weight dextrans. The term "micelle" as used in conjunction with this invention, relates to submicroscopic particles which are thermodynamically stable in solution, and particularly in anticoagulated whole mammalian blood. The micelles are formed from two or more macromolecules, at least one of which forms a positively charged exterior surface on the micelles, which positive surface charge serves to neutralize or reduce the strength of the repulsive forces exerted by the zeta potential of the erythrocytes. The macromolecule micelles are preferably formed in an aqueous solution such as saline, which is admixed with an anticoagulated whole blood sample. Certain cationic ultra-high molecular weight substances such as PVP can also be used alone or in combination with others of the aforesaid reagents.

This invention also contemplates adding to the blood sample erythrocyte-specific binding agents, such as anti-glycophorin antibodies or lectins along with the aforesaid macromolecular reagents, when necessary to produce a clarified erythrocyte-granulocyte interface in a centrifuged sample of anticoagulated whole blood.

It is therefore an object of this invention to provide a method for treating an anticoagulated whole blood sample so as to enhance the tendency of the erythrocytes in the blood sample to form rouleaux or other aggregates.

It is a further object of this invention to provide a method of the character described wherein erythrocyte repulsion resulting from the zeta potential of the erythrocytes is suppressed.

It is an additional object of this invention to provide a method of the character described wherein the blood sample is mixed with a reagent solution containing one or more high molecular weight cationic substances which enhance erythrocyte aggregation to a degree that they will gravimetrically layer out by mean erythrocyte density when the blood sample is centrifuged.

It is another object of this invention to provide a method of the character described wherein the blood sample may also be mixed with a binding agent which will agglutinate the erythrocytes so that they will gravimetrically layer out by mean erythrocyte density when the blood sample is centrifuged.

These and other objects and advantages of the invention will become more readily apparent to those skilled in the art from the following detailed description of preferred embodiments of the invention when taken in conjunction with the accompanying drawings, in which:

### Brief Description of the Drawings

FIG. 1 is a perspective view of a transparent sample tube containing a centrifuged blood sample and a cell layer-elongating insert;
FIG. 2 is a schematic illustration of the manner in which erythrocytes repel each other due to their negative surface charge;
FIG. 3 is a schematic representation of a micelle formed from a combination of dextran and Polybrene, which micelle possesses a positive surface charge;
FIG. 4 is a schematic representation of the manner in which the addition of micelles to a blood sample allows adjacent erythrocytes to more closely aggregate by reducing or neutralizing the effect of the zeta potential; and
FIG. 5 is a schematic representation of the manner in which the addition of micelles to a blood sample serves to reduce or neutralize the strength with which erythrocytes repel each other so as to allow erythrocyte-specific binding agents in the blood sample to agglutinate erythrocytes to each other.

### Best Mode For Carrying Out The Invention

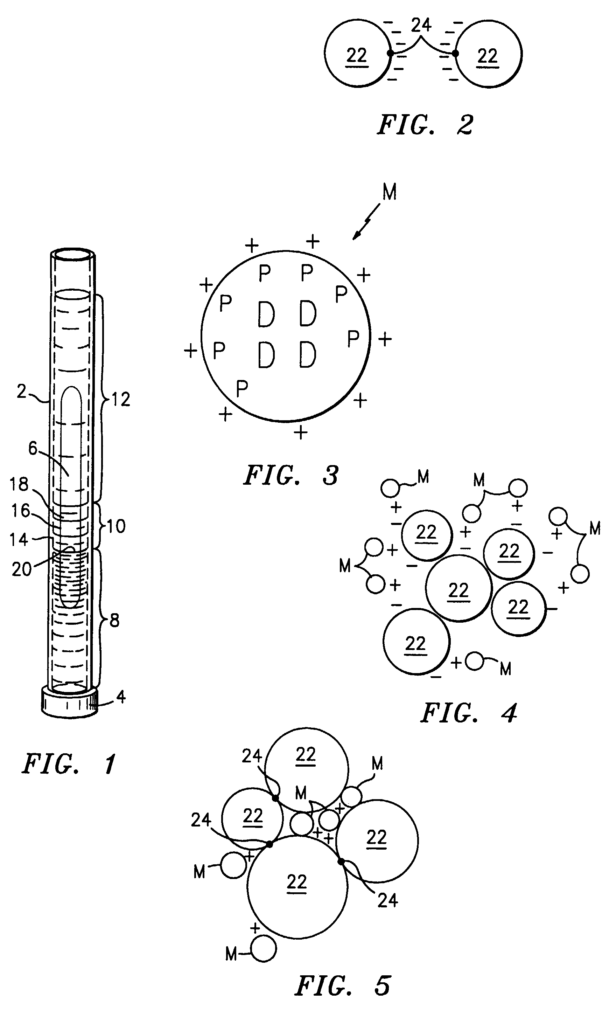

Referring now to the drawings, there is shown in FIG. 1 a blood sampling and testing assembly of the type preferred in practicing the method of this invention. The assembly includes a transparent tube 2 into which an anticoagulated whole blood sample is drawn and centrifuged. The tube 2 contains a blood cell layer-elongating insert 6 which is generally cylindrical in shape, and has a specific gravity such that it will float in the erythrocyte layer 8 of a centrifuged anticoagulated whole blood sample in the tube 2. The bottom of the tube 2 will be closed by a cap 4 to enable centrifugation of the sample in the tube 2. When the blood sample is centrifuged in the tube 2, the constituents thereof gravimetrically form separate layers according to specific gravity. The heaviest layer is the erythrocyte layer 8; followed by the buffy coat layer 10; and the plasma layer 12. The buffy coat 10 consists of three primary component layers, i.e., the granulocyte layer 14, the lymphocyte/monocyte layer 16, and the platelet layer 18. The interface 20 between the erythrocyte layer 8 and the granulocyte layer 14 is clarified and sharply defined by the addition to the blood sample of the combination of high molecular weight cationic molecules, with, or in certain cases, without the addition of erythrocyte binding agents.

FIG. 2 is a schematic representation of the manner in which adjacent erythrocytes 22 in a sample of anticoagulated whole blood tend to repel each other due to their zeta potential surface negative charge, to an extent which will interfere with the ability of erythrocyte-specific binding agents 24 on adjacent cells 22 to agglutinate the cells 22 to each other. It will be understood that for binding agent-induced agglutination of adjacent erythrocytes 22 to occur, a binding agent unit 24 must be able to attach to the surface membrane of each of the erythrocytes 22. FIG. 2 illustrates that, in certain cases, the gap between adjacent erythrocytes 22 which is caused by zeta potential repulsion forces is sufficiently large to prevent binding agent units 24 from agglutinating adjacent erythrocytes 22.

FIG. 3 is a schematic representation of a micelle formed in accordance with this invention. The micelle is denoted generally by the letter M, and is formed from a cluster of macromolecules. One operable micelle structure M includes a plurality of core dextran molecules D, and an outer surface formed from a plurality of Polybrene molecules P. The dextran molecules D provide the desired volume for the micelle M; and the Polybrene molecules P provide the desired positive surface charge on the micelles M, as illustrated in FIG. 3.

FIG. 4 is a schematic representation of the manner in which the high molecular weight cationic micelles promote rouleaux and other forms of erythrocyte aggregation in anticoagulated bovine whole blood. In FIG. 4, the numeral 22 denotes individual erythrocyte cells and the letter M denotes the cationic high molecular weight rouleaux or erythrocyte aggregation-enhancing micelles or cationic macromolecules, such as PVP. It will be noted that the mix of cationic micelles or macromolecules M are distributed among the erythrocyte cells 22 and are operative to bring the cells 22 in closer proximity with each other. With the inclusion of the cationic high molecular weight micelles or macromolecules M, the aggregation of the cells 22 is enhanced.

FIG. 5 is a schematic representation of the manner in which the micelles or macromolecules M promote the formation of rouleaux, or other erythrocyte aggregates, to a degree necessary to allow the erythrocyte binding agent units 24 to agglutinate the erythrocytes 22 together.

One specific application of the above-identified technology relates to the q.b.c.a. of a centrifuged sample of anticoagulated whole bovine blood. Bovine blood has proven so far to be incapable of analysis by using the q.b.c.a. technique. The reason for this fact is the unusually high zeta potential exhibited by bovine erythrocytes.

Erythrocytes taken from anticoagulated bovine whole blood samples, which were allowed to sediment, which were not combined with high molecular weight micelles or macromolecules of the types described above, when examined under a microscope showed zero clumping, aggregation or rouleaux formation. Anticoagulated whole bovine blood was admixed with antibodies; and separately with high molecular weight cationic molecules; and then centrifuged in the q.b.c.a. tube-and-insert paraphernalia described above. The result was a centrifuged mixture which displayed an indistinct and murky erythrocyte/granulocyte interface which did not allow a clinically acceptable measurement of the white cell and platelet differential counts by means of the q.b.c.a. technique.

In contrast, when saline solutions of combinations of high molecular weight micelles, and in certain cases macromolecules, were admixed with the anticoagulated bovine whole blood, significant erythrocyte aggregation or rouleaux formation was observed microscopically. Particularly effective high molecular weight reagents include micelles formed from Polybrene and dextran; micelles formed from polybrene and PVP; and cationic PVP macromolecules. It was noted that bovine blood samples that were admixed with saline solutions containing the aforesaid reagents and were then allowed to sediment, resulted in the formation of numerous erythrocyte aggregates, or rouleaux, when viewed under a microscope. Anticoagulated bovine blood samples that had been admixed with saline solutions containing essentially only one or the other of the Polybrene and dextran reagents did not demonstrate any significant erythrocyte aggregation or rouleaux formation.

Following the precepts of this invention, a sample of anticoagulated bovine whole blood was admixed with a saline solution containing micelles formed from Polybrene and dextran. The mixture was centrifuged in the prior art q.b.c.a. tube-and-insert paraphernalia, and the resultant gravimetrically separated mixture displayed a clarified and well defined erythrocyte-granulocyte interface sufficient to provide a clinically acceptable measurement of the differential white cell counts, as well as hematocrit, hemoglobin and platelet counts, by means of the q.b.c.a. technique.

A first preferred solution for use in testing bovine blood by means of the q.b.c.a. technology consists of micelles formed from about 65 mg Polybrene and about 65 mg dextran/ml saline. The aforesaid admixture provides sufficient erythrocyte aggregation so as to allow accurate differential white cell and platelet counts to be made, and also allows accurate hematocrit and hemoglobin readings to be made in a centrifuged sample of anticoagulated bovine whole blood. Operable concentrations of blood to saline solution are in the range of about 2:1 to about 10:1.

A second preferred solution for use in testing bovine blood by means of the q.b.c.a. technology consists of micelles formed from about 65 mg polybrene and about 65 mg PVP/ml saline. The aforesaid admixture provides sufficient erythrocyte aggregation so as to allow accurate differential white cell and platelet counts to be made, and also allows accurate hematocrit and hemoglobin readings to be made in a centrifuged sample of anticoagulated bovine whole blood. Operable concentrations of blood to the saline solution are in the range of about 2:1 to about 10:1.

A third preferred solution for use in testing bovine blood by means of the q.b.c.a. technology consists of 65 mg macromolecules of PVP per ml saline. The aforesaid admixture also provides sufficient erythrocyte aggregation so as to allow accurate differential white cell counts to be made, and also allows accurate hematocrit and hemoglobin, and platelet count readings to be made in a centrifuged sample of anticoagulated bovine whole blood. Operable concentrations of blood to the saline solution are in the range of about 2:1 to about 10:1.

The aforesaid determinations of optimum micelle and macromolecule saline solution concentrations for use in the analysis of bovine blood are the work of Messers. John Roche, Paul Weiss and Travis Waldron of IDEXX Laboratories, Inc..

Another specific application of the above-identified technology relates to the q.b.c.a. of a centrifuged sample of anticoagulated whole human blood which has exhibited the streaming problem. As noted above, human blood which has demonstrated excessive streaming has proven so far to be very difficult to analyze by using the q.b.c.a. technique. The reason for this fact is unknown.

Samples of anticoagulated whole human blood which are known to exhibit the "streaming" effect, were admixed with-a saline solution of Polybrene and dextran micelles, and then centrifuged in the q.b.c.a. paraphernalia shown in FIG. 1 and described above. The result was a centrifuged mixture which improved the streaming problem but not sufficiently for clinically acceptable measurements of the white cell differential counts, or hematocrit and hemoglobin by means of the q.b.c.a. technique. Likewise, admixtures of streaming blood and only one of the high molecular weight reagents did not improve the streaming effect, nor did admixtures of streaming blood and anti-glycophorin antibody.

When the streaming blood samples were admixed with a combination of Polybrene and dextran micelles, along with the anti-glycophorin antibody, improved granulocyte-erythrocyte separation was achieved and the interface between the aforesaid two cell layers in a meaningful percentage of centrifuged streaming blood samples was significantly clarified. About half of the streaming blood samples tested, when combined with the Polybrene-dextran micelle solution plus the anti-glycophorin antibody, demonstrated a significant improvement in the degree of cell separation which enabled the use of the q.b.c.a. procedure to procure accurate differential white cell and platelet counts, as well as hematocrit and hemoglobin readings.

In particular, streaming human blood samples were admixed with anti-glycophorin antibody plus a saline solution containing micelles formed from about 65 mg and about 65 mg dextran/ml saline. The blood-reagent mixture was then allowed to sediment resulting in the formation of numerous erythrocyte aggregates, or rouleaux, when viewed under a microscope. A preferred concentration range of blood to erythrocyte aggregating agent solution for analyzing streaming human blood samples is from about 2:1 to about 10:1. Similar results may be obtained in a more convenient manner by forming a dry blood-soluble coating of the binding agent and/or the erythrocyte-aggregating agents in appropriate concentrations on the wall of the blood sampling tubes. The polybrene-PVP micelles and the PVP macromolecules described above, when used in conjunction with an erythrocyte binding agent, should also prove to be useful in achieving a reduction in the streaming problem.

It will be readily appreciated that the addition to blood of combinations of cationic high molecular weight micellar compounds or cationic macromolecular agents alone, or in certain cases, in combination with erythrocyte-specific binding agents, will significantly increase the ability of erythrocytes to aggregate, form rouleaux, and be agglutinated. Blood samples which are thus modified can therefore be more accurately analyzed by means of the q.b.c.a. technique. In particular, samples of treated anticoagulated bovine whole blood can be analyzed using the aforesaid technique, and treated streaming human blood samples can also be better analyzed using the aforesaid technique.

Since many changes and variations of the disclosed embodiments of the invention may be made without departing from the inventive concept, it is not intended to limit the invention otherwise than as required by the appended claims.

## Claims

1. A method for forming a well defined and distinct interface between a layer of erythrocytes and a layer of granulocytes in a sample of centrufuged anticoagulated whole blood, said method comprising the steps of:
a) forming a blood-micelle mixture by admixing the blood sample with a quantity of micelles which are formed from at least two different high molecular weight particles, which micelles possess a positive surface charge; and
b) centrifuging the blood-micelle mixture in a transparent tube.

2. The method of Claim 1 comprising the further step of admixing said blood-micelle mixture with an erythrocyte-specific binding agent prior to said centrifugation step.

3. The method of Claim 1 where said blood sample is bovine blood.

4. The method of Claim 1 wherein said high molecular weight particles are agents selected from the group consisting of polybrene, protamine sulfate, cationic antibiotics, polyethylene glycol (PEG), hydroxydiethyl starch (HES), polyvinyl pyrrolidone (PVP), and dextrans.

5. The method of Claim 1 wherein said micelles are formed from a mixture of polybrene and dextran.

6. The method of Claim 1 wherein said micelles are formed from a mixture of polybrene and PVP.

7. A method for forming a well defined and distinct interface between a layer of bovine erythrocytes and a layer of bovine granulocytes in a sample of centrufuged anticoagulated bovine whole blood, said method comprising the steps of:
a) providing a bovine blood-micelle mixture by admixing the bovine blood sample with a quantity of micelles which are formed from at least two different high molecular weight particles, which micelles possess a positive surface charge; and
b) centrifuging the bovine blood-micelle mixture in a transparent tube.

8. A method for forming a well defined and distinct interface between a layer of erythrocytes and a layer of granulocytes in a sample of centrufuged anticoagulated whole blood, said method comprising the steps of:
a) providing a blood-micelle mixture by admixing the blood sample with a quantity of micelles which are formed from clusters of dextran and polybrene; and
b) centrifuging the blood-micelle mixture in a transparent tube.

9. A method for forming a well defined and distinct interface between a layer of erythrocytes and a layer of granulocytes in a sample of centrufuged anticoagulated whole blood, said method comprising the steps of:
a) providing a blood-micelle mixture by admixing the blood sample with a quantity of micelles comprising clusters of polyvinyl pyrrolidone and polybrene; and
b) centrifuging the blood-micelle mixture in a transparent tube.

10. A method for reducing the magnitude of the repulsion force resulting from erythrocytes 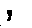 zeta potential in a sample of anticoagulated whole blood, said method comprising the step of forming an anticoagulated blood-micelle mixture by admixing the blood sample with a quantity of micelles which are formed from at least two different high molecular weight particles, which micelles possess a positive surface charge.

11. The method of Claim 10 wherein said high molecular weight particles include dextran and polybrene.

12. The method of Claim 10 wherein said high molecular weight particles include polyvinyl pyrrolidone and polybrene.

13. A method for forming a well defined and distinct interface between a layer of erythrocytes and a layer of granulocytes in a sample of centrufuged anticoagulated whole blood, said method comprising the steps of:
a) providing a blood-micelle mixture by admixing the blood sample with a quantity of micelles comprising clusters of polyvinyl pyrrolidone and polybrene; and
b) centrifuging the blood-micelle mixture in a transparent tube.

14. A method for reducing the magnitude of the repulsion force resulting from erythrocytes 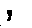 zeta potential in a sample of anticoagulated whole blood, said method comprising the step of forming an anticoagulated blood-micelle mixture by admixing the blood sample with a quantity of micelles which are formed from at least two different high molecular weight particles, which micelles possess a positive surface charge.

15. A method for forming a well defined and distinct interface between a layer of erythrocytes and a layer of granulocytes in a sample of centrufuged anticoagulated whole blood, said method comprising the steps of:
a) providing a mixture of anticoagulated whole blood and a cationic macromolecular agent; and
b) centrifuging the blood-agent mixture in a transparent tube.

16. The method of Claim 15 wherein said macromolecular agent is polyvinyl pyrrolidone.

17. The method of Claim 15 wherein said blood sample is bovine blood.

18. A method for reducing the magnitude of the repulsion force resulting from erythrocytes 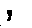 zeta potential in a sample of anticoagulated whole blood, said method comprising the step of forming an anticoagulated blood-cationic macromolecular agent mixture by admixing the blood sample with a quantity of cationic macromolecules.

19. The method of Claim 18 wherein said macromolecular agent is polyvinyl pyrrolidone.

20. The method of Claim 18 wherein said blood sample is bovine blood.

21. A solution of micelles in an aqueous medium, said micelles being formed from clusters of macromolecular agents, said agents including a core macromolecular constituent and a surface macromolecular constituent, said surface macromolecular constituent providing the micelles with a surface positive charge.

22. The solution of Claim 21 wherein said aqueous medium is a saline solution.

23. The solution of Claim 21 wherein said micelles are formed from clusters of dextran and polybrene.

24. The solution of Claim 21 wherein said micelles are formed from clusters of polyvinyl pyrrolidone and polybrene.

25. A blood analysis assembly comprising a transparent blood sampling tube, said tube having a bore for receiving and containing a blood sample, said tube bore being coated with a dry reagent which is soluble in blood and which reagent includes macromolecular agents which are capable of forming micelles when in solution, and which are operative to suppress the zeta potential of erythrocytes in anticoagulated whole blood contained in the tube.
